(19) Europäisches Patentamt
European Patent Office
Office européen des brevets

(11) Publication number: **0 011 363**
**B1**

# (12) EUROPEAN PATENT SPECIFICATION

(45) Date of publication of patent specification: **07.04.82**

(51) Int. Cl.³: **C 07 F 9/65, A 01 N 57/08**

(21) Application number: **79302026.4**

(22) Date of filing: **28.09.79**

## (54) Pyrazole phosphates and phosphonates, preparation thereof and use thereof as insecticides.

(30) Priority: **02.10.78 US 947803**

(43) Date of publication of application:
**28.05.80 Bulletin 80/11**

(45) Publication of the grant of the patent:
**07.04.82 Bulletin 82/14**

(84) Designated Contracting States:
**AT BE CH DE FR GB IT NL SE**

(56) References cited:
**DE - A - 2 049 692**
**FR - A - 1 331 721**
**US - A - 4 094 974**

(73) Proprietor: **STAUFFER CHEMICAL COMPANY**
**Westport Connecticut 06880 (US)**

(72) Inventor: **Fancher, Llewellyn W.**
**22 Valley View Drive**
**Orinda, California 94563 (US)**

(74) Representative: **Smith, Sydney et al,**
**High Holborn House 52/54 High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Pyrazole phosphates and phosphonates, preparation thereof and use thereof as insecticides

This invention relates to a pyrazole phosphates and phosphonates, preparation thereof and use thereof as insecticides.

The present invention provides a compound corresponding to the following general formula:

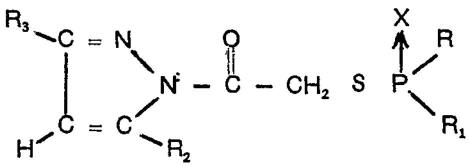

wherein

X represents oxygen; R represents $C_1$—$C_6$ alkoxy; $R_1$ represents $C_1$—$C_6$ alkoxy; $R_2$ represents methyl; and $R_3$ represents methyl;

or wherein

X represents oxygen; R represents $C_1$—$C_6$ alkoxy; $R_1$ represents $C_1$—$C_6$ alkoxy; $R_2$ represents hydrogen; and $R_3$ represents methyl;

or wherein

X represents sulphur; R represents $C_1$—$C_6$ alkoxy; $R_1$ represents $C_1$—$C_6$ alkoxy; $R_2$ represents methyl; and $R_3$ represents methyl;

or wherein

X represents sulphur; R represents $C_1$—$C_6$ alkyl; $R_1$ represents $C_1$—$C_6$ alkoxy; $R_2$ represents methyl; and $R_3$ represents methyl;

or wherein

X represents sulphur; R represents $C_1$—$C_6$ alkyl; $R_1$ represents $C_1$—$C_6$ alkoxy; $R_2$ represents hydrogen; and $R_3$ represents methyl;

or wherein

X represents sulphur; R represents $C_1$—$C_6$ alkoxy; $R_1$ represents $C_1$—$C_6$ alkoxy; $R_2$ represents hydrogen; and $R_3$ represents methyl;

or wherein

X represents sulphur; R represents $C_1$—$C_6$ alkyl; $R_1$ represents $C_1$—$C_6$ alkoxy; $R_2$ represents methyl; and $R_3$ represents phenyl;

or wherein

X represents sulphur; R represents $C_1$—$C_6$ alkoxy; $R_1$ represents $C_1$—$C_6$ alkoxy; $R_2$ represents methyl; and $R_3$ represents phenyl;

or wherein

X represents oxygen; R represents $C_1$—$C_6$ alkoxy; $R_1$ represents $C_1$—$C_6$ alkoxy; $R_2$ represents methyl; and $R_3$ represents phenyl.

By way of further exemplification, the present invention relates to a number of preferred compounds according to the above-defined general formula:

wherein

X represents oxygen; R represents ethoxy; $R_1$ represents ethoxy; $R_2$ represents methyl; and $R_3$ represents methyl;

or wherein

X represents oxygen; R represents ethoxy; $R_1$ represents ethoxy; $R_2$ represents hydrogen; and $R_3$ represents methyl;

or wherein

X represents sulphur; R represents ethoxy; $R_1$ represents ethoxy; $R_2$ represents methyl; and $R_3$ represents methyl;

or wherein

X represents sulphur; R represents methoxy; $R_1$ represents methoxy; $R_2$ represents methyl; and $R_3$ represents methyl;

or wherein

X represents sulphur; R represents ethyl; $R_1$ represents ethoxy; $R_2$ represents methyl; and $R_3$ represents methyl;

or wherein

X represents sulphur; R represents ethyl; $R_1$ represents isopropoxy; $R_2$ represents methyl; and $R_3$ represents methyl;

or wherein

X represents sulphur; R represents ethyl; $R_1$ represents isobutoxy; $R_2$ represents methyl; and $R_3$ represents methyl;

or wherein

X represents sulphur; R represents ethyl; $R_1$ represents ethoxy; $R_2$ represents hydrogen; and $R_3$ represents methyl;

or wherein

X represents sulphur; R represents ethyl; $R_1$ represents isopropoxy; $R_2$ represents hydrogen; and $R_3$ represents methyl;

or wherein

X represents sulphur; R represents ethyl; $R_1$ represents isobutoxy; $R_2$ represents hydrogen; and $R_3$ represents methyl;

or wherein

X represents sulphur; R represents methoxy; $R_1$ represents methoxy; $R_2$ represents hydrogen; and $R_3$ represents methyl;

or wherein

X represents sulphur; R represents ethoxy; $R_1$ represents ethoxy; $R_2$ represents hydrogen; and $R_3$ represents methyl;

or wherein

X represents sulphur; R represents ethyl; $R_1$ represents ethoxy; $R_2$ represents methyl; and $R_3$ represents phenyl;

or wherein

X represents sulphur; R represents ethyl; $R_1$ represents isopropoxy; $R_2$ represents methyl; and $R_3$ represents phenyl;

or wherein

X represents sulphur; R represents ethyl; $R_1$ represents isobutoxy; $R_2$ represents methyl; and $R_3$ represents phenyl;

or wherein

X represents sulphur; R represents ethoxy; $R_1$ represents ethoxy; $R_2$ represents methyl; and $R_3$ represents phenyl;

or wherein

X represents sulphur; R represents methoxy; $R_1$ represents methoxy; $R_2$ represents methyl; and $R_3$ represents phenyl;

or wherein

X represents oxygen; R represents ethoxy; $R_1$ represents ethoxy; $R_2$ represents methyl; and $R_3$ represents phenyl.

The present invention also provides a process for the preparation of such a compound characterised in that it comprises reacting an appropriate chloroacetyl-pyrazole with an appropriate phosphoric or phosphonic acid.

The present invention further provides an insecticidal composition characterised in that it comprises such a compound and an agronomically acceptable carrier or diluent.

Such a composition generally comprises not more than 80%, by weight, of the above-mentioned active compound.

The present invention further provides a method of controlling insects characterised in that it comprises applying to the insects and/or to the habitats thereof and/or to the feedstuffs thereof such a compound or such a composition.

As indicated above, the present compounds are useful as insecticides when used in an insecticidally effective amount. By the term "insecticidally effective amount" is meant the amount of a compound which, when applied in a conventional manner, with kill or substantially injure a significant portion of the insects.

Regarding the preparation of the present compounds, for example, pyrazoles as intermediates may be prepared by the cyclo-condensation of a dicarbonyl compound, such as acetyl acetone, with hydrazine (see *Organic Synthesis,* Vol. 31, page 43).

Chloroacetyl pyrazoles may be conveniently prepared by the following general reaction. The pyrazole may be chloroacetylated by the reaction of chloroacetic anhydride with a pyrazole in an inert solvent, such as tetrahydrofuran. Chloroacetic anhydride is convenient, but chloroacetyl chloride and a base are also operable. The chloroacetyl pyrazole may be isolated by treatment with water, filtration of the resulting solid, followed by drying.

The product may be prepared by reacting a chloroacetyl pyrazole with a phosphoric or phosphonic acid, neutralized with a base, such as triethylamine, or a preformed salt of the acid, such as the sodium, potassium or ammonium salt, in the presence of an inert solvent. Dimethyl formamide is the preferred solvent, since the chloroacetyl pyrazole has limited solubility in other non-reactive solvents. The reaction may be carried out at or near room temperature to prevent side reactions which may occur at higher temperatures. The product may be isolated by dilution with a water-insoluble solvent, such as benzene or toluene. This may be followed by washing with water, drying (anhydrous magnesium sulphate), filtering and evaporation of the solvent.

Various pyrazole phosphates and phosphonates may be prepared by using the above-exemplified reactants and procedures. The following Examples are further illustrations of the preparation of compounds according to the present invention.

Example 1

Preparation of 1-0,0-diethylphosphorodithioylacetyl-3,5-dimethylpyrazole.

0,0-diethylphosphorodithioic acid, 3.72 grams (0.02 mole) $[(C_2H_5O)_2P(S)SH]$, was dissolved in 25 millilitres of dimethyl formamide. This acid solution was neutralized with triethylamine, 2.01 grams (0.02 mole), while cooling the vessel. To this solution was added 1-chloroacetyl-3,5-dimethyl-pyrazole, 2.59 grams (0.015 mole). The reaction mixture was stirred at ambient temperature for 4 hours. At that time, the reaction mixture was diluted with benzene, 150 millilitres, followed by washing with dilute sodium chloride solution, two 100 millilitre portions. The organic phase was separated and dried over magnesium sulphate, filtered and the organic solvent removed under vacuum. There was obtained 4.71 grams of the title compound, a light yellow liquid, $n_D^{30}$1.5355. Product exhibited solubility in acetone. NMR analysis confirmed the structure.

Example 2

Preparation of 1-chloroacetyl-3,5-dimethylpyrazole

In tetrahydrofuran, 50 millilitres, there was slurried 3,5-dimethylpyrazole, 19.2 grams (0.2 mole). To this slurry was added at one time chloroacetic anhydride, 37.6 grams (0.22 mole). A solution resulted. The temperature rose from 17°C to 34°C. The solution was allowed to stand for 1 hour at ambient temperature, followed by reflux on a steam bath for 5 minutes. After standing overnight, the reaction mixture was poured into cold water, 50 millilitres. A solid material precipitated. To hasten the reaction of chloroacetic anhydride with water, the mixture was heated to 50°C on a steam bath. After standing for 15 minutes, the solution was cooled to 15°C, filtered and washed with cold water. After washing twice more with *n*-hexane, the product was dried at 50°C for 1 hour and then at 40°C. There was obtained 30.1 grams of the title compound as a fine needle-like white solid, m.p. 72—75°C, soluble in acetone. The structure was confirmed by nuclear magnetic resonance spectroscopy.

Preparation of 1-ethyl, O-ethylphosphonodithioylacetyl-3,5-dimethylpyrazole.

In a similar reaction to that described in Example 1, ethyl, O-ethylphosphonodithioic acid, 3.40 grams (0.02 mole), was dissolved in dimethyl formamide, 25 millilitres, with stirring. The solution was cooled and neutralized with triethylamine, 2.01 grams (0.02 mole), at a temperature below 30°C. To this solution was added 1-chloroacetyl-3,5-dimethyl-pyrazole, 2.59 grams (0.15 mole). This mixture was stirred at ambient temperature for 4 hours. After 4 hours, the reaction mixture was diluted with benzene, 180 millilitres, washed with dilute sodium chloride solution, two 100 millilitre portions, and dried over magnesium sulphate. After filtering and evaporating the organic solvent under vacuum, there was obtained 4.54 grams of the title compound as a yellow liquid, $n_D^{30}$1.5482. The structure was confirmed by nuclear magnetic resonance spectroscopy.

Example 3

Preparation of 1-ethyl, O-isobutyl-phosphonodithioylacetyl-3,5-dimethylpyrazole.

In dimethyl formamide, 25 millilitres, ethyl, O-isobutyl-phosphono-dithioic acid, 3.96 grams (0.02 mole), was dissolved. This neutralized with triethylamine, 2.01 grams (0.02 mole), with cooling below 30°C. There was added to this solution 1-chloroacetyl-3,5-dimethylpyrazole, 2.59 grams (0.015 mole). The reaction mixture was stirred at ambient temperature for 4 hours. At the end of this period, the reaction mixture was diluted with benzene, 150 millilitres and washed with dilute sodium chloride solution, two 150 millilitre portions. The organic portion was separated and dried over magnesium sulphate, filtered and the organic solvent evaporated under vacuum. There was obtained a yellow liquid, 4.70 grams of the title compound, $n_D^{30}$ 1.5357. The structure was confirmed by nuclear magnetic resonance spectroscopy.

Example 4

Preparation of 1-0,0-diethylphosphonomonothioylacetyl-3,5-dimethylpyrazole.

In dimethyl formamide, 25 millilitres, potassium 0,0-diethylphosphonomonothioate, 4.16 grams (0.02 mole), was dissolved. To this solution was added 1-chloroacetyl-3,5-dimethylpyrazole, 2.59 grams (0.015 mole), and stirred at room temperature for 4 hours. The reaction mixture was diluted with benzene, 100 millilitres, and washed with dilute sodium chloride solution, 100 millilitre portions. After separation of the organic phase, it was dried over magnesium sulphate, filtered and the organic solvent evaporated under vacuum. There was obtained 4.5 grams of the title compound as an amber coloured liquid, $n_D^{30}$1.5075. The structure was confirmed by nuclear magnetic resonance spectroscopy.

Example 5

Preparation of 1-N-0,0-diethylphosphorodithioylacetyl-3-phenyl-5-methyl-pyrazole.

By a similar procedure to that described in Example 3, 90 percent O-ethyl phosphonondithioic acid, 3.88 grams (0.019 mole), was dissolved in dimethyl formamide, 25 millilitres, and neutralized with triethylamine, 1.9 grams (0.019 mole), with cooling below 25°C. To this solution was added 1-chloroacetyl-3-phenyl-5-methyl-pyrazole, 3.28 grams (0.014 mole), and the reaction mixture stirred at ambient temperature for 4 hours. At the end of this period, the reaction mixture was diluted with

toluene, 100 millilitres, and washed with dilute sodium chloride solution, two 75 millilitre portions. After separation, the organic portion was dried over magnesium sulphate, filtered and the organic solvent evaporated *in vacuo*. There was obtained 5.26 grams of the title compound, $n_D^{30}$ 1.5774. The structure was confirmed by nuclear magnetic resonance spectroscopy.

Example 6

Preparation of 1-N-O,O-diethylphosphoromonothioylacetyl-3-phenyl-5-methyl-pyrazole.

In dimethyl formamide, 25 millilitres, potassium O,O-diethylphosphoromonothioate, 2.8 grams (0.0135 mole), was dissolved. To this solution was added 1-chloroacetyl-3-phenyl-5-methyl-pyrazole, 2.04 grams (0.0087 mole). The reaction mixture was stirred at ambient temperature for 4 hours. At the end of this period, the mixture was diluted with toluene, 100 millilitres, and washed with water, two 75 millilitre portions. After separation, the organic phase was dried over magnesium sulphate, filtered and evaporated *in vacuo*. There was obtained 2.8 grams of the title compound, an amber coloured liquid, $n_D^{30}$ 1.5615. The structure was confirmed by nuclear magnetic resonance spectroscopy.

The following is a Table of representative and illustrative compounds that may be prepared according to the aforementioned general procedures. Compound numbers have been assigned to the compounds and are used for identification throughout the remainder of the specification.

(The compounds prepared in the above Examples correspond to compounds 1,3,5,9,14 and 18, respectively).

TABLE I

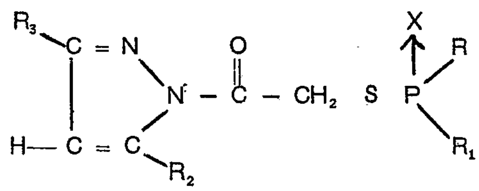

| Compound Number | X | R | $R_1$ | $R_2$ | $R_3$ | m.p. or $n_D^{30}$ |
|---|---|---|---|---|---|---|
| 1 | S | $OC_2H_5$ | $OC_2H_5$ | $CH_3$ | $CH_3$ | 1.5355 |
| 2 | S | $OCH_3$ | $OCH_3$ | $CH_3$ | $CH_3$ | 1.5496 |
| 3 | S | $C_2H_5$ | $OC_2H_5$ | $CH_3$ | $CH_3$ | 1.5482 |
| 4 | S | $C_2H_5$ | O-*i*-$C_3H_7$ | $CH_3$ | $CH_3$ | 1.5405 |
| 5 | S | $C_2H_5$ | O-*i*-$C_4H_9$ | $CH_3$ | $CH_3$ | 1.5357 |
| 6 | S | $C_2H_5$ | $OC_2H_5$ | H | $CH_3$ | 1.5551 |
| 7 | S | $C_2H_5$ | O-*i*-$C_3H_7$ | H | $CH_3$ | 1.5434 |
| 8 | S | $C_2H_5$ | O-*i*-$C_4H_9$ | H | $CH_3$ | 1.5330 |
| 9 | O | $OC_2H_5$ | $OC_2H_5$ | $CH_3$ | $CH_3$ | 1.5075 |
| 10 | S | $OCH_3$ | $OCH_3$ | H | $CH_3$ | 1.5480 |
| 11 | O | $OC_2H_5$ | $OC_2H_5$ | H | $CH_3$ | 1.5055 |
| 12 | S | $OC_2H_5$ | $OC_2H_5$ | H | $CH_3$ | 1.5370 |
| 13 | S | $C_2H_5$ | $OC_2H_5$ | $CH_3$ | $C_6H_5$ | 86—90°C |
| 14 | S | $OC_2H_5$ | $OC_2H_5$ | $CH_3$ | $C_6H_5$ | 1.5774 |
| 15 | S | $C_2H_5$ | O-*i*-$C_3H_7$ | $CH_3$ | $C_6H_5$ | 77—80°C |
| 16 | S | $C_2H_5$ | O-*i*-$C_4H_9$ | $CH_3$ | $C_6H_5$ | 1.5752 |

TABLE I (continued)

| Compound Number | X | R | $R_1$ | $R_2$ | $R_3$ | m.p. or $n_D^{30}$ |
|---|---|---|---|---|---|---|
| 17 | S | $OCH_3$ | $OCH_3$ | $CH_3$ | $C_6H_5$ | 1.5980 |
| 18 | O | $OC_2H_5$ | $OC_2H_5$ | $CH_3$ | $C_6H_5$ | 1.5615 |
| 19 | S | $OC_2H_5$ | $OC_2H_5$ | $CH_3$ | H | |
| 20 | O | $OC_2H_5$ | $OC_2H_5$ | $CH_3$ | H | |
| 21 | S | $C_2H_5$ | O-*i*-$C_3H_7$ | $CH_3$ | H | |
| 22 | O | $C_2H_5$ | O-*i*-$C_3H_7$ | $CH_3$ | H | |
| 23 | S | $OC_2H_5$ | $OC_2H_5$ | H | $CH_3$ | |
| 24 | O | $OC_2H_5$ | $OC_2H_5$ | H | $CH_3$ | |
| 25 | S | $C_2H_5$ | O-*i*-$C_4H_9$ | $CH_3$ | $C_6H_5$ | |
| 26 | O | $C_2H_5$ | O-*i*-$C_4H_9$ | $CH_3$ | $C_6H_5$ | |
| 27 | S | $OC_2H_5$ | $OCH_3$ | H | $C_6H_5$ | |
| 28 | O | $OCH_3$ | $OCH_3$ | H | $C_6H_5$ | |
| 29 | S | $OC_2H_5$ | $OC_2H_5$ | H | H | |
| 30 | S | $C_2H_5$ | $OC_2H_5$ | H | H | |
| 31 | O | $OC_2H_5$ | $OC_2H_5$ | H | H | |

## INSECTICIDAL EVALUATION TESTS

It is to be noted that the term "insect" is used herein in its broad common usage to include spiders, mites, ticks and similar pests which are not in the strict biological sense classified as insects. The term "insect" is used herein to refer not only to those small invertebrate animals belonging mostly to the class Insecta, comprising six-legged usually winged forms, such as beetles, bugs, bees and flies, but also to other allied classes of arthropods whose members are wingless and usually have more than six legs, such as spiders, mites, ticks, centipedes and wood lice.

The following insect species were used in evaluation tests for insecticidal activity according to the following procedures.

(A.) Housefly [*Musca domestica* (L.)] — HF

The test compound is diluted in acetone and an aliquot is pipetted onto the bottom of 55 × 15 millimetre aluminium dish. To ensure even spreading on the bottom of the dish, 1 millilitre of acetone containing 0.02% peanut oil is added. After all the solvent was evaporated, the dish is placed in a circular cardboard cage containing 25 one-day-old female houseflies. The cage is covered on the bottom with "Cellophane" and the top with tulle netting and contains a sugar-water saturated cotton plug for maintenance of the flies. Mortality is recorded after 48 hours. The primary screening level for this test is 100 micrograms of the test compound per 25 female houseflies.

(B.) Lygus bug [*Lygus hesperus* (Knight)] — LB

The test compound is dissolved in a 50—50 acetone-water solution. 2 millilitres of the solution are sprayed by means of a hand spray gun into a circular cardboard cage covered on the bottom with "Cellophane" and the top with tulle netting containing one string bean pod and ten adult lygus bugs. Percent mortality is recorded after 48 hours. The primary screening level for this test is 0.05%, by weight, of the test compound in the acetone-water solution.

(C.) Direct Spray Assay on Black Bean Aphid
[*Aphis fabae* (Scop.)] — BA

A nasturtium plant *(Tropaeolum sp.)*, approximately 5 centimetres tall, is transplanted into sandy loam soil in a 7 or 8 cm (3 inch) clay pot and infested with from 25 to 50 black bean aphids of mixed

ages. 24 hours later, the plant is sprayed, to the point of runoff, with a 50—50 acetone-water solution of the test chemical. The treated plant is maintained in the greenhouse and mortality is recorded after 3 days. The primary screening level of this test is 0.05%, by weight, of the test compound in the acetone-water solution.

(D.) Black Bean Aphid Systemic Evaluation Test —BAS

This test evaluates the root absorption and upward translocation of the candidate systemic compound. The bean aphid (BA) [*Aphis fabae* (Scop.)] was employed in the test for systemic activity. Young nasturtium plants were used as the host plants for the bean aphid. The host plants were transplanted into 0.45 kg (1 pound) of soil that had been treated with the candidate compound. Immediately after planting in the treated soil, the plants were infested with the aphids. Concentrations of toxicant in the soil ranged from 10 ppm downward until an LD-50 value was obtained. Mortality was recorded after 72 hours. The percentage of kill of the test species was determined by comparison with control plants placed in untreated soil. The LD-50 values were calculated.

(E.) Direct Spray Assay on Green Peach Aphid [*Myzus persicae* (Sulzer)] — GPA

A radish plant (*Rhaphanus sativus*), approximately 2 centimetres tall, is transplanted into sandy loam soil in a 7 or 8 cm (3 inch) clay pot and infested with from 25 to 50 green peach aphids of mixed ages. 24 hours later, the plant is sprayed, to the point of runoff, with a 50—50 acetone-water solution of the test compound. The treated plant is maintained in a greenhouse and mortality is recorded after 3 days. The primary screening level for this test is 0.05%, by weight, of the test compound in the acetone-water solution.

(F.) German Cockroach [*Blatella germanica* (Linné)] — GR

Test compounds are diluted in a 50—50 acetone-water solution. 2 millilitres of the solution are sprayed by means of a hand spray gun into circular cardboard cages containing 10 one-month-old German cockroach nymphs. The test cages are covered on the bottom with "Cellophane" and the top with tulle netting. Percent mortality is recorded 7 days later. Test concentrations range from 0.1% down to that at which approximately 50% mortality occurs.

(G.) Saltmarsh Caterpillar (*Estigmene acrea* (Drury)] — SMC

A test solution is prepared by dissolving the test compound in a 50—50 acetone-water solution. A section of a curly dock (*Rumex crispus* leaf, approximately 2.5 centimetres wide and 4 centimetres long, is immersed in the test solution for from 2 to 3 seconds then placed on a wire screen to dry. The dried leaf was then placed in a Petri dish containing a moistened piece of filter paper and infested with 5 second-instar saltmarsh caterpillar larvae. Mortality of the larvae is recorded 48 hours later. If surviving larvae are still present, a piece of synthetic media is added to the dish and the larvae are observed for an additional 5 days in order to detect delayed effects of the test compound. The primary screening level for this test is 0.05% by weight, of the test compound in the solution.

(H.) Cabbage Looper [*Trichoplusia ni* (Hübner)]—CL

The procedure for cabbage looper larvae is similar to that used for saltmarsh caterpillar larvae, except that a cotyledon of hyzini squash (*Calabacita abobrinha)* of approximately the same size as the curly dock leaf section is used in place of the latter. The primary screening level for this test is 0.1%, by weight, of the test compound in the solution.

(I.) Tobacco Budworm [*Heliothis virescens* (F.)]—TBW

Larvae of the tobacco budworm are used in this test in a procedure similar to that used for saltmarsh caterpillar larvae, except that a Romaine lettuce (*Latucs sativa*) leaf section of approximately the same size as the curly dock leaf section is used in place of the latter. The primary screening level for this test is 0.1%, by weight, of the test compound in the solution.

(J.) Southern House Mosquito [*Culex pipiens quinquefasciatus* (Say)] — MOS

Insecticidal activity is determined using third instar larvae of the mosquito *Culex pipiens quinquefasciatus.* 10 larvae are placed in a 170 g. (6 ounce) paper cup containing 100 millilitres of an aqueous solution of the test chemical. The treated larvae are stored at 21°C (70°F), and 48 hours later the mortality is recorded. Test concentrations range from 0.5 ppm down to that at which approximately 50% mortality occurs.

(K.) Two-spotted Mite [*Tetranychus urticae* (Koch)] — 2SM

A pinto bean plant (*Phaseolus sp.*) approximately 10 centimetres tall is transplanted into sandy loam soil in a 7 or 8 cm (3 inch) clay pot and infested with two-spotted mites of mixed ages and sexes. 24 hours later the infested plants are inverted and dipped for from 2 to 3 seconds in a 50—50 acetone-water solution of the test compound. The treated plant is maintained in a greenhouse for 7 days. Mortality is then determined for both the adult mites and the nymphs hatching from eggs which were on the plants at the time of treatment. The primary screening level for this test is 0.05%, by weight, of the test compound in the acetone — water solution.

Table II below is a summary of the results of tests performed on the representative compounds listed in Table I. These test results are expressed as $LD_{50}$ values, which represent the dose of test compound which was lethal to 50% of the insect population in the test. The entries in Table II were obtained as follows:

For a particular insect, each compound was initially tested at the primary screening level. Those

compounds showing less than 50% kill at this level are represented in the Table by the primary screening level preceded by a "greater than" sign (<). Those compounds showing approximately 50% kill are represented by the primary screening level alone. Those compounds showing greater than 50% kill were subjected to further testing at successively lower levels, until the level was found at which approximately 50% kill was achieved. The latter level is listed as the $LD_{50}$ for this group.

The primary screening level in each of the above tests was selected for purposes of convenience only and none of the figures in the Table are to be understood as representing the highest level at which a viable test for insecticidal activity may be conducted. (Dashes are used in Table II where no tests were performed.)

TABLE II

INSECTICIDAL ACTIVITY — APPROXIMATE $LD_{50}$ VALUES

| Compound Number | HF | BA | BAS | GPA | GR | LB |
|---|---|---|---|---|---|---|
| 1 | 35 | .03 | >10 | .03 | >.1 | >.05 |
| 2 | 35 | .03 | >10 | .05 | >.1 | >.05 |
| 3 | 19 | .0005 | >10 | .002 | .01 | >.05 |
| 4 | 21 | .0005 | >10 | .003 | .05 | <.05 |
| 5 | 20 | .0003 | 2 | .0005 | .01 | >.05 |
| 6 | >100 | .005 | >10 | .03 | >.1 | >.05 |
| 7 | >100 | .005 | 1 | .03 | >.1 | >.05 |
| 8 | 100 | .002 | 10 | .005 | >.1 | >.05 |
| | >100 | | | | | |
| 9 | >10 | .0008 | >10 | .05 | .1 | >.05 |
| | | >.05 | | | | |
| 10 | >100 | >.005 | — | — | — | — |
| 11 | 100 | >.05 | — | — | — | — |
| 12 | >100 | >.05 | — | — | — | — |
| | >100 | | | | | |
| 13 | >10 | .005 | >10 | — | — | — |
| 14 | >100 | .005 | >10 | — | — | — |
| 15 | 100 | .003 | >10 | .002 | >.1 | >.05 |
| 16 | >100 | .01 | >10 | .05 | >.1 | >.05 |
| 17 | >100 | >.05 | — | — | — | — |
| 18 | >100 | >.05 | — | — | — | — |

TABLE II (continued)

| Compound Number | SMC | CL | TBW | MOS | 2SM Adults | 2SM Eggs | 2SM Sys |
|---|---|---|---|---|---|---|---|
| 1 | >.05 | >.1 | >.1 | 1 | ≤.05 | >.05 | >10 |
| 2 | >.05 | >.1 | >.1 | >1 | >.05 | >.05 | — |
| 3 | .05 | >.1 | .1 | >1 | <.05 | <.05 | >10 |
| 4 | >.05 | >.1 | .1 | >1 | >.05 | >.05 | — |
| 5 | .05 | .01 | .02 | >1 | .05 | <.05 | >10 |
| 6 | >.05 | >.1 | >.1 | >1 | <.05 | <.05 | >10 |
| 7 | >.05 | >.1 | >.1 | >1 | >.05 | .05 | >10 |
| 8 | >.05 | >.1 | >.1 | >1 | .05 | <.05 | >10 |
| 9 | >.05 | >.05 | >.05 | >1 | >.05 | >.05 | >10 |
| 10 | — | — | >.05 | >1 | >.05 | >.05 | — |
| 11 | — | — | >.05 | >1 | >.05 | .05 | — |
| 12 | — | — | >.05 | >1 | >.05 | >.05 | — |
| 13 | — | — | >.05 | >1 | <.05 >.005 | >.05 | >10 |
| 14 | — | — | >.05 | >1 | >.05 | — | |
| 15 | >.05 | >.05 | >.05 | >1 | <.05 >.005 | <.05 >.005 | >10 |
| 16 | >.05 | >.05 | .05 | >1 | <.05 >.005 | <.05 >.005 | — |
| 17 | — | — | >.05 | >1 | >.05 | >.05 | — |
| 18 | — | — | >.05 | >1 | >.05 | >.05 | — |

In general terms, the amount of active compound or composition which is considered to be insecticidally effective is that amount which, when applied to the insect pest, habitat or feedstuff, will kill or substantially injure a significant portion residing or feeding thereof. The active compounds according to the present invention may be employed either as the sole insecticide component of the compositions or as one of a mixture of compounds in the composition having similar utility. Furthermore, the present compositions need not be active as such. The purposes of the present invention will be fully served by a composition which is rendered active by external influences, such as light, or by physiological action occurring when the preparation is ingested or penetrates into the body of the pest.

The precise manner in which the insecticidal compounds according to the present invention are used in a particular instance will be readily apparent to those skilled in the art. Generally, the active compound will be used as a component of a liquid composition, for example, an emulsion, suspension or aerosol spray. While the concentration of the active compound in the present composition may vary within rather wide limits, ordinarily, the pesticide composition will comprise less than 80.0 percent, by weight, of the composition.

The compositions according to the present invention are generally applied to the pest, pest habitat or feedstuff to be treated in an agricultural formulation which also comprises an agronomically acceptable carrier. By the term "agronomically acceptable carrier "is meant a substance which may be used to dissolve, disperse or diffuse an active compound in the composition without impairing the

effectiveness of the compound and which by itself has no detrimental effect on the soil, equipment, crops or agronomic environment. The compositions according to the present invention may be either solid or liquid formulations or solutions. For example, the compounds may be formulated as wettable powders, emulsifiable concentrates, granulates or dusts. In such formulations, the compounds are extended with a liquid or solid carrier and, when desired, suitable surfactants are incorporated.

It is usually desirable to include adjuvants, such as wetting agents, spreading agents, dispersing agents, stickers and adhesives, in accordance with conventional agricultural practices.

For the preparation of emulsifiable concentrates, one or more of the active compounds may be dissolved in one or more organic solvents, such as benzene, toluene, xylene, methylated naphthalene, corn oil, pine oil, o-dichlorobenzene, isophorone, cyclohexane and methyl oleate, or mixtures thereof, together with an emulsifying agent which permits dispersion in water. Suitable emulsifiers include, for example, the ethylene oxide derivatives of alkylphenols or long chain alcohols, mercaptans, carboxylic acids and reactive amines and partially esterified polyhydric alcohols. Solvent-soluble sulphates or sulphonates, such as the alkaline earth salts or amine salts of alkylbenzene sulphonates and the fatty alcohol sodium sulphates, having surface-active properties may be used as emulsifiers either alone or in conjunction with an ethylene oxide reaction product.

Wettable powders suitable for spraying may be prepared by admixing one or more of the active compounds with a finely divided solid, such as clays, inorganic silicates and carbonates and silicas, and by incorporating wetting agents, sticking agents and/or dispersing agents in such mixtures. The concentration of active ingredients in such compositions is usually from 20 to 80%, by weight. A dispersing agent may constitute from 0.5 to 3% of the composition and a wetting agent may constitute from 0.1 to 5% of the composition.

Dusts may be prepared by mixing the composition according to the present invention with one or more finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing from 20 to 80%, by weight, of the active ingredients are commonly made and are subsequently diluted to from 1 to 10% use concentration.

The compounds according to the present invention may be applied as sprays by methods commonly employed, such as conventional high gallonage hydraulic sprays, low gallonage sprays, air-blast spray, aerial sprays and dusts. For low volume applications, a solution of the active compounds is usually used. The dilution and rate of application will usually depend upon various factors, such as the type of equipment employed, the method of application and area to be treated.

For some applications, it may be useful to add one or more other insecticides to the compositions according to the present invention. Examples of other insecticides which may be incorporated to provide additional advantages include: parathion, methyl parathion, malathion, carbaryl, methomyl dicofol, monocrotophos and chlordimeform. Other pesticides, including fungicides and plant bactericides, may also be included in the compositions according to the present invention.

**Claims for the contracting States: BE, CH, DE, FR, GB, IT, NL, SE**

1. A compound corresponding to the following general formula:

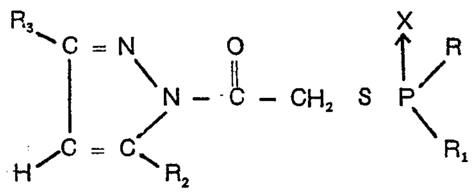

wherein

X represents oxygen or sulphur;
R represents $C_1$—$C_6$ alkyl or $C_1$—$C_6$ alkoxy;
$R_1$ represents $C_1$—$C_6$ alkoxy;
$R_2$ represents hydrogen or methyl; and
$R_3$ represents hydrogen, methyl or phenyl.

2. A compound according to claim 1

wherein

X represents oxygen; R represents $C_1$—$C_6$ alkoxy; $R_1$ represents $C_1$—$C_6$ alkoxy; $R_2$ represents methyl; and $R_3$ represents methyl;

or wherein

X represents oxygen; R represents $C_1$—$C_6$ alkoxy; $R_1$ represents $C_1$—$C_6$ alkoxy; $R_2$ represents hydrogen; and $R_3$ represents methyl;

or wherein

X represents sulphur; R represents $C_1$—$C_6$ alkoxy; $R_1$ represents $C_1$—$C_6$ alkoxy; $R_2$ represents

methyl; and $R_3$ represents methyl;
or wherein

X represents sulphur; R represents $C_1$—$C_6$ alkyl; $R_1$ represents $C_1$—$C_6$ alkoxy; $R_2$ represents methyl; and $R_3$ represents methyl;
or wherein

X represents sulphur; R represents $C_1$—$C_6$ alkyl; $R_1$ represents $C_1$—$C_6$ alkoxy; $R_2$ represents hydrogen; and $R_3$ represents methyl;
or wherein

X represents sulphur; R represents $C_1$—$C_6$ alkoxy; $R_1$ represents $C_1$—$C_6$ alkoxy; $R_2$ represents hydrogen; and $R_3$ represents methyl;
or wherein

X represents sulphur; R represents $C_1$—$C_6$ alkyl; $R_1$ represents $C_1$—$C_6$ alkoxy; $R_2$ represents methyl; and $R_3$ represents phenyl;
or wherein

X represents sulphur; R represents $C_1$—$C_6$ alkoxy; $R_1$ represents $C_1$—$C_6$ alkoxy; $R_2$ represents methyl; and $R_3$ represents phenyl;
or wherein

X represents oxygen; R represents $C_1$—$C_6$ alkoxy; $R_1$ represents $C_1$—$C_6$ alkoxy; $R_2$ represents methyl; and $R_3$ represents phenyl.

3. A compound according to claim 1 or claim 2 wherein

X represents oxygen; R represents ethoxy; $R_1$ represents ethoxy; $R_2$ represents methyl; and $R_3$ represents methyl;
or wherein

X represents oxygen; R represents ethoxy; $R_1$ represents ethoxy; $R_2$ represents hydrogen; and $R_3$ represents methyl;
or wherein

X represents sulphur; R represents ethoxy; $R_1$ represents ethoxy; $R_2$ represents methyl; and $R_3$ represents methyl;
or wherein

X represents sulphur; R represents methoxy; $R_1$ represents methoxy; $R_2$ represents methyl; and $R_3$ represents methyl;
or wherein

X represents sulphur; R represents ethyl; $R_1$ represents ethoxy; $R_2$ represents methyl; and $R_3$ represents methyl;
or wherein

X represents sulphur; R represents ethyl; $R_1$ represents isopropoxy; $R_2$ represents methyl; and $R_3$ represents methyl;
or wherein

X represents sulphur; R represents ethyl; $R_1$ represents isobutoxy; $R_2$ represents methyl; and $R_3$ represents methyl;
or wherein

X represents sulphur; R represents ethyl; $R_1$ represents ethoxy; $R_2$ represents hydrogen; and $R_3$ represents methyl;
or wherein

X represents sulphur; R represents ethyl; $R_1$ represents isopropoxy; $R_2$ represents hydrogen; and $R_3$ represents methyl;
or wherein

X represents sulphur; R represents ethyl; $R_1$ represents isobutoxy; $R_2$ represents hydrogen; and $R_3$ represents methyl;
or wherein

X represents sulphur; R represents methoxy; $R_1$ represents methoxy; $R_2$ represents hydrogen; and $R_3$ represents methyl;
or wherein

X represents sulphur; R represents ethoxy; $R_1$ represents ethoxy; $R_2$ represents hydrogen; and $R_3$ represents methyl;
or wherein

X represents sulphur; R represents ethyl; $R_1$ represents ethoxy; $R_2$ represents methyl; and $R_3$ represents phenyl;
or wherein

X represents sulphur; R represents ethyl; $R_1$ represents isopropoxy; $R_2$ represents methyl; and $R_3$ represents phenyl;
or wherein

X represents sulphur; R represents ethyl; $R_1$ represents isobutoxy; $R_2$ represents methyl; and $R_3$ represents phenyl;
or wherein

X represents sulphur; R represents ethoxy; $R_1$ represents ethoxy; $R_2$ represents methyl; and $R_3$ represents phenyl;
or wherein
X represents sulphur; R represents methoxy; $R_1$ represents methoxy; $R_2$ represents methyl; and $R_3$ represents phenyl;
or wherein
X represents oxygen; R represents ethoxy; $R_1$ represents ethoxy; $R_2$ represents methyl; and $R_3$ represents phenyl.

4. A process for the preparation of a compound according to claim 1 characterised in that it comprises reacting an appropriate chloroacetyl-pyrazole with an appropriate phosphoric or phosphonic acid.

5. An insecticidal composition characterised in that it comprises a compound according to any of claims 1 to 3 and an agronomically acceptable carrier or diluent.

6. A composition according to claim 5 characterised in that it comprises not more than 80%, by weight, of a compound according to any of claims 1 to 3.

7. A method of controlling insects characterised in that it comprises applying to the insects and/or to the habitats thereof and/or to the feedstuffs thereof a compound according to any of claims 1 to 3 or a composition according to claim 5 or claim 6.

**Patentansprüche für die Vertragsstaaten: BE, CH, DE, FR, GB, IT, NL, SE**

1. Verbindungen der folgenden allgemeinen Formel

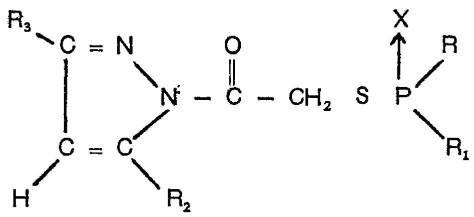

worin
X Sauerstoff oder Schwefel;
R $C_{1-6}$-Alkyl oder $C_{1-6}$-Alkoxy;
$R_1$ $C_{1-6}$-Alkoxy;
$R_2$ Wasserstoff oder Methyl; und
$R_3$ Wasserstoff, Methyl oder Phenyl
bedeuten.

2. Verbindungen nach Anspruch 1, dadurch gekennzeichnet, daß
X Sauerstoff; R $C_{1-6}$-Alkoxy; $R_1$ $C_{1-6}$-Alkoxy; $R_2$ Methyl; und $R_3$ Methyl;
oder
X Sauerstoff; R $C_{1-6}$-Alkoxy; $R_1$ $C_{1-6}$-Alkoxy; $R_2$ Wasserstoff; und $R_3$ Methyl;
oder
X Schwefel; R $C_{1-6}$-Alkoxy; $R_1$ $C_{1-6}$-Alkoxy; $R_2$ Methyl; und $R_3$ Methyl;
oder
X Schwefel; R $C_{1-6}$-Alkyl; $R_1$ $C_{1-6}$-Alkoxy; $R_2$ Methyl; und $R_3$ Methyl;
oder
X Schwefel; R $C_{1-6}$-Alkyl; $R_1$ $C_{1-6}$-Alkoxy; $R_2$ Wasserstoff; und $R_3$ Methyl;
oder
X Schwefel; R $C_{1-6}$-Alkoxy; $R_1$ $C_{1-6}$-Alkoxy; $R_2$ Wasserstoff; und $R_3$ Methyl;
oder
X Schwefel; R $C_{1-6}$-Alkyl; $R_1$ $C_{1-6}$-Alkoxy; $R_2$ Methyl; und $R_3$ Phenyl;
oder
X Schwefel; R $C_{1-6}$-Alkoxy; $R_1$ $C_{1-6}$-Alkoxy; $R_2$ Methyl; und $R_3$ Phenyl;
oder
X Sauerstoff; R $C_{1-6}$-Alkoxy; $R_1$ $C_{1-6}$-Alkoxy; $R_2$ Methyl; und $R_3$ Phenyl
bedeuten.

3. Verbindungen nach einem der Ansprüche 1 oder 2, dadurch gekennzeichnet, daß
X Sauerstoff; R Äthoxy; $R_1$ Äthoxy; $R_2$ Methyl; und $R_3$ Methyl;
oder
X Sauerstoff; R Äthoxy; $R_1$ Äthoxy; $R_2$ Wasserstoff; und $R_3$ Methyl;
oder
X Schwefel; R Äthoxy; $R_1$ Äthoxy; $R_2$ Methyl; und $R_3$ Methyl;
oder

X Schwefel; R Methoxy; $R_1$ Methoxy; $R_2$ Methyl; und $R_3$ Methyl;
oder
X Schwefel; R Äthyl; $R_1$ Äthoxy; $R_2$ Methyl; und $R_3$ Methyl;
oder
X Schwefel; R Äthyl; $R_1$ Isopropoxy; $R_2$ Methyl; und $R_3$ Methyl;
oder
X Schwefel; R Äthyl; $R_1$ Isobutoxy; $R_2$ Methyl; und $R_3$ Methyl;
oder
X Schwefel; R Äthyl; $R_1$ Äthoxy; $R_2$ Wasserstoff; und $R_3$ Methyl;
oder
X Schwefel; R Äthyl; $R_1$ Isopropoxy; $R_2$ Wasserstoff; und $R_3$ Methyl;
oder
X Schwefel; R Äthyl; $R_1$ Isobutoxy; $R_2$ Wasserstoff; und $R_3$ Methyl;
oder
X Schwefel; R Methoxy; $R_1$ Methoxy; $R_2$ Wasserstoff; und $R_3$ Methyl;
oder
X Schwefel; R Äthoxy; $R_1$ Äthoxy; $R_2$ Wasserstoff; und $R_3$ Methyl;
oder
X Schwefel; R Äthyl; $R_1$ Äthoxy; $R_2$ Methyl; und $R_3$ Phenyl;
oder
X Schwefel; R Äthyl; $R_1$ Isopropoxy; $R_2$ Methyl; und $R_3$ Phenyl;
oder
X Schwefel; R Äthyl; $R_1$ Isobutoxy; $R_2$ Methyl; und $R_3$ Phenyl;
oder
X Schwefel; R Äthoxy; $R_1$ Äthoxy; $R_2$ Methyl; und $R_3$ Phenyl;
oder
X Schwefel; R Methoxy; $R_1$ Methoxy; $R_2$ Methyl; und $R_3$ Phenyl;
oder
X Sauerstoff; R Äthoxy; $R_1$ Äthoxy; $R_2$ Methyl; und $R_3$ Phenyl
bedeuten.

4. Verfahren zur Herstellung einer Verbindung nach Anspruch 1, dadurch gekennzeichnet, daß man ein geeignetes Chloracetylpyrazol mit einer geeigneten Phosphor- oder Phosphonsäure umsetzt.

5. Insektenvernichtende Zubereitung, dadurch gekennzeichnet, daß sie eine Verbindung nach einem der Ansprüche 1 bis 3 und einen agronomisch annehmbaren Träger oder ein Verdünnungsmittel enthält.

6. Zubereitung nach Anspruch 5, dadurch gekennzeichnet, daß sie nicht mehr als 80 Gew.% einer Verbindung nach einem der Ansprüche 1 bis 3 enthält.

7. Verfahren zur Kontrolle von Insekten, dadurch gekennzeichnet, daß man auf die Insekten und/oder ihre Umgebung und/oder ihr Futter eine Verbindung nach einem der Ansprüche 1 bis 3 oder eine Zubereitung nach einem der Ansprüche 5 oder 6 anwendet.

**Revendications pour les Etats contractants: BE, CH, DE, FR, GB, IT, NL, SE**

1. Composé correspondant à la formule générale ci-dessous:

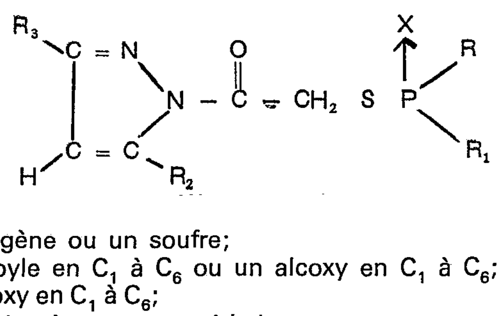

où
X représente un oxygène ou un soufre;
R représente un alcoyle en $C_1$ à $C_6$ ou un alcoxy en $C_1$ à $C_6$;
$R_1$ représente un alcoxy en $C_1$ à $C_6$;
$R_2$ représente un hydrogène ou un méthyle; et
$R_3$ représente un hydrogène, un méthyle ou un phényle.

2. Composé selon la revendication 1, où
X représente un oxygène; R représente un alcoxy en $C_1$ à $C_6$; $R_1$ représente un alcoxy en $C_1$ à $C_6$; $R_2$ représente un méthyle; et $R_3$ représente un méthyle;
X représente un oxygène; R représente un alcoxy en $C_1$ à $C_6$; $R_1$ représente un alcoxy en $C_1$ à $C_6$; $R_2$ représente un hydrogène; et $R_3$ représente un méthyle;
ou où
X représente un soufre; R représente un alcoxy en $C_1$ à $C_6$; $R_1$ représente un alcoxy en $C_1$ à $C_6$; $R_2$

représente un méthyle; et $R_3$ représente un méthyle;

ou où

X représente un soufre; R représente un alcoyle en $C_1$ à $C_6$; $R_1$ représente un alcoxy en $C_1$ à $C_6$; $R_2$ représente un méthyle; et $R_3$ représente un méthyle;

ou où

X représente un soufre; R représente un alcoyle en $C_1$ à $C_6$; $R_1$ représente un alcoxy en $C_1$ à $C_6$; $R_2$ représente un hydrogène; et $R_3$ représente un méthyle;

ou où

X représente un soufre; R représente un alcoxy en $C_1$ à $C_6$; $R_1$ représente un alcoxy en $C_1$ à $C_6$; $R_2$ représente un hydrogène; et $R_3$ représente un méthyle;

ou où

X représente un soufre; R représente un alcoyle en $C_1$ à $C_6$; $R_1$ représente un alcoxy en $C_1$ à $C_6$; $R_2$ représente un méthyle, et $R_3$ représente un phényle;

ou où

X représente un soufre; R représente un alcoxy en $C_1$ à $C_6$; $R_1$ représente un alcoxy en $C_1$ à $C_6$; $R_2$ représente un méthyle; et $R_3$ représente un phényle;

ou où

X représente un oxygène, R représente un alcoxy en $C_1$ à $C_6$; $R_1$ représente un alcoxy en $C_1$ à $C_6$; $R_2$ représente un méthyle; et $R_3$ représente un phényle.

3. Composé selon la revendications 1 ou la revendication 2 où

X représente un oxygène; R représente un éthoxy; $R_1$ représente un éthoxy; $R_2$ représente un méthyle; et $R_3$ représente un méthyle;

ou où

X représente un oxygène; R représente un éthoxy; $R_1$ représente un éthoxy; $R_2$ représente un hydrogène; et $R_3$ représente un méthyle;

ou où

X représente un soufre; R représente un éthoxy; $R_1$ représente un éthoxy; $R_2$ représente un méthyle; et $R_3$ représente un méthyle;

ou où

X représente un soufre; R représente un méthoxy; $R_1$ représente un méthoxy; $R_2$ représente un méthyle; et $R_3$ représente un méthyle;

ou où

X représente ou soufre; R représente un éthyle; $R_1$ représente un éthoxy; $R_2$ représente un méthyle; et $R_3$ représente un méthyle;

ou où

X représente un soufre; R représente un éthyle; $R_1$ représente un isopropoxy; $R_2$ représente un méthyle; et $R_3$ représente un méthyle;

ou où

X représente un soufre; R représente un éthyle; $R_1$ représente un isobutoxy; $R_2$ représente un méthyle; et $R_3$ représente un méthyle;

ou où

X représente un soufre; R représente un éthyle; $R_1$ représente un éthoxy; $R_2$ représente un hydrogène; et $R_3$ représente un méthyle;

ou où

X représente un soufre; R représente un éthyle; $R_1$ représente un isopropoxy; $R_2$ représente un hydrogène; et $R_3$ représente un méthyle;

ou où

X représente un soufre; R représente un éthyle; $R_1$ représente un isobutoxy; $R_2$ représente un hydrogène; et $R_3$ représente un méthyle;

ou où

X représente un soufre; R représente un méthoxy; $R_1$ représente un méthoxy; $R_2$ représente un hydrogène; et $R_3$ représente un méthyle;

ou où

X représente un soufre; R représente un éthoxy; $R_1$ représente un éthoxy; $R_2$ représente un hydrogène; et $R_3$ représente un méthyle;

ou où

X représente un soufre; R représente un éthyle; $R_1$ représente un éthoxy; $R_2$ représente un méthyle; et $R_3$ représente un phényle;

ou où

X représenteun soufre; R représente un éthyle; $R_1$ représente un isopropoxy; $R_2$ représente un méthyle; et $R_3$ représente un phényle;

ou où

X représente un soufre; R représente un éthyle; $R_1$ représente un isobutoxy; $R_2$ représente un méthyle; et $R_3$ représente un phényle;

ou où

X représente un soufre; R représente un éthoxy; $R_1$ représente un éthoxy; $R_2$ représente un méthyle; et $R_3$ représente un phényle;
ou où

X représente un soufre: R représente un méthoxy; $R_1$ représente un méthoxy; $R_2$ représente un méthyle; et $R_3$ représente un phényle;
ou où

X représente un oxygène, R représente un éthoxy; $R_1$ représente un éthoxy; $R_2$ représente un méthyle; et $R_3$ représente un phényle.

4. Procédé de préparation d'un composé selon la revendication 1 caractérisé en ce qu'il implique de faire réagir un chloroacétyl-pyrazole approprié avec un acide phosphorique ou phosphonique approprié.

5. Composition insecticide caractérisée en ce qu'elle comprend un composé selon l'une quelconque des revendications 1 à 3 et un support ou diluant agronomiquement acceptable.

6. Composition selon la revendication 5 caractérisé en ce qu'elle ne comprend pas plus de 80%, en poids, d'un composé selon l'une quelconque des revendications 1 à 3.

7. Procédé de lutte contre les insectes caractérisé en ce qu'il implique d'appliquer aux insectes et/ou à leur habitat et/ou à leur nourriture un composé selon l'une quelconque des revendications 1 à 3 ou une composition selon la revendication 5 ou la revendication 6.

**Claims for the contracting State: AT**

1. A process for the preparation of a compound corresponding to the following general formula:

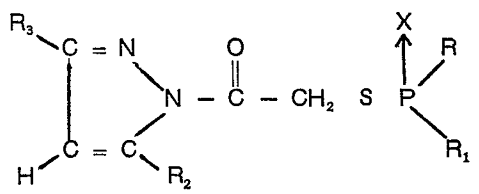

wherein

X represents oxygen or sulphur;
R represents $C_1$—$C_6$ alkyl or $C_1$—$C_6$ alkoxy;
$R_1$ represents $C_1$—$C_6$ alkoxy;
$R_2$ represents hydrogen or methyl; and
$R_3$ represents hydrogen, methyl or phenyl;

characterised in that it comprises reacting an appropriate chloroacetyl-pyrazole with an appropriate phosphoric or phosphonic acid.

2. An insecticidal composition characterised in that it comprises a compound prepared by a process according to claim 1 and an agronomically acceptable carrier or diluent.

3. A composition according to claim 2 characterised in that it comprises not more than 80%, by weight, of a compound prepared by a process according to claim 1.

4. A method of controlling insects characterised in that it comprises applying to the insects and/or to the habitats thereof and/or to the feedstuffs thereof a compound prepared by a process according to claim 1 or a composition according to claim 2 or claim 3.

**Patentansprüche für den Vertragsstaat: AT**

1. Verfahren zur Herstellung einer Verbindung entsprechend der folgenden allgemeinen Formel

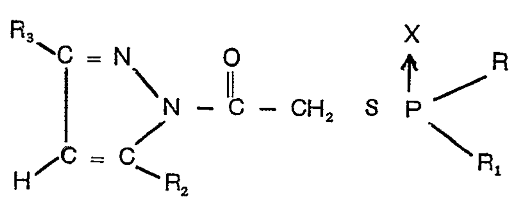

worin

X Sauerstoff oder Schwefel;
R $C_{1-6}$-Alkyl oder $C_{1-6}$-Alkoxy;
$R_1$ $C_{1-6}$-Alkoxy;
$R_2$ Wasserstoff oder Methyl; und
$R_3$ Wasserstoff, Methyl oder Phenyl

bedeuten, dadurch gekennzeichnet, daß man ein geeignetes Chloracetylpyrazol mit einer geeigneten Phosphor- oder Phosphonsäure umsetzt.

2. Insektenvernichtende Zubereitung, dadurch gekennzeichnet, daß sie eine gemäß dem Verfahren nach Anspruch 1 hergestellte Verbindung und einen agronomisch annehmbaren Träger oder Verdünnungsmittel enthält.

3. Zubereitung nach Anspruch 2, dadurch gekennzeichnet, daß sie nicht mehr als 80 Gew.% einer Verbindung, die nach dem Verfahren gemäß Anspruch 1 hergestellt worden ist, enthält.

4. Verfahren zur Kontrolle von Insekten, dadurch gekennzeichnet, daß man auf die Insekten und/oder ihre Umgebung und/oder ihr Futter eine Verbindung, die nach dem Verfahren von Anspruch 1 erhalten worden ist, oder eine Zubereitung nach einem der Ansprüche 2 oder 3 anwendet.

**Revendications pou l'Etat contractant: AT**

1. Procédé de préparation d'un composé correspondant à la formule générale ci-dessous:

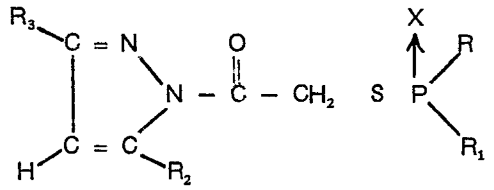

où

X représente un oxygène ou un soufre;

R représente un alcoyle en $C_1$ à $C_6$ ou un alcoxy en $C_1$ à $C_6$;

$R_1$ représente un alcoxy en $C_1$ à $C_6$;

$R_2$ représente un hydrogène ou un méthyle; et

$R_3$ représente un hydrogène, un méthyle ou un phényle;

caractérisé en ce qu'il implique de faire réagir un chloroacétyl-pyrazole approprié avec un acide phosphorique ou phosphonique approprié.

2. Composition insecticide caractérisée en ce qu'elle comprend un composé préparé par un procédé selon la revendication 1 et un support ou diluant agronomiquement acceptable.

3. Composition selon la revendication 2 caractérisée en ce qu'elle ne comprend pas plus de 80%, en poids, d'un composé préparé par un procédé selon la revendication 1.

4. Procédé de lutte contre les insectes caractérisé en ce qu'il implique d'appliquer aux insectes et/ou à leur habitat et/ou à leur nourriture un composé préparé par un procédé selon la revendication 1 ou une composition selon la revendication 2 ou la revendication 3.